# EUROPÄISCHE PATENTANMELDUNG

(11) **EP 4 817 650 A1**
(43) Veröffentlichungstag der Anmeldung: **07.10.2026**
(21) Anmeldenummer: 26169021.8
(22) Anmeldetag: 30.03.2026
(51) Int. Cl.: A61M 1/16

(54) **EXTRAKORPORALE BLUTBEHANDLUNGSMASCHINE UND VERFAHREN FÜR EINEN TEMPERATURABHÄNGIGEN MASCHINENPROZESS EINER EXTRAKORPORALEN BLUTBEHANDLUNGSMASCHINE**

(30) Priorität: 03.04.2025 DE 102025113136
(71) Anmelder: B. Braun Avitum AG, 34212 Melsungen (DE)
(72) Erfinder: WAGNER, Andre, 34119 Kassel (DE); SCHELLHASE, Meik, 36179 Bebra (DE)
(74) Vertreter: Winter, Brandl - Partnerschaft mbB

(57) **Zusammenfassung**

Die Offenbarung betrifft eine extrakorporale Blutbehandlungsmaschine zur Durchführung eines temperaturabhängigen Maschinenprozesses (H, T) mit einem Fluidkreislauf (5), einer Heizeinrichtung (28), um ein Fluid des Fluidkreislaufs (5) zur Ausführung des Maschinenprozesses (H, T) aufzuheizen, an Abschnitten (30, 38, 50, 58) des Fluidkreislaufs (5) angeordneten Temperatur-Erfassungseinheiten (32, 46, 52, 54, 60, 62), um eine jeweilige Abschnitts-Temperatur (T1, T2, T3, T4, T5, T6) des Fluids zu erfassen, einer hydraulische Fördereinrichtung (36, 48), um das Fluid wahlweise zu fördern oder nicht zu fördern, und einer Steuereinheit (68), in der eine Korrelation (70) einer Laufzeit (tp) des wenigstens einen Maschinenprozesses (H, T) in Abhängigkeit von einer Umgebungstemperatur (Tu) abgelegt ist, wobei über die Steuereinheit (68) ein Mittelwert (Tx) der erfassten Abschnitts-Temperaturen (T1, T2, T3, T4, T5, T6) bildbar ist, in Abhängigkeit des Mittelwertes (Tx) in der Korrelation (70) die Umgebungstemperatur (Tu) bestimmbar ist, und mittels der Korrelation (70) in Abhängigkeit der Umgebungstemperatur (Tu) eine Laufzeit (tp) des Maschinenprozesses (H, T) bestimmbar ist. Die Offenbarung betrifft zudem ein Verfahren für einen temperaturabhängigen Maschinenprozess einer extrakorporalen Blutbehandlungsmaschine.

## Beschreibung

### Technisches Gebiet

Die vorliegende Offenbarung betrifft eine extrakorporale Blutbehandlungsmaschine, insbesondere eine Dialysemaschine, sowie ein Verfahren für einen temperaturabhängigen Maschinenprozess einer extrakorporalen Blutbehandlungsmaschine, insbesondere einer Dialysemaschine.

In der chronischen Dialyse werden extrakorporale Blutbehandlungsmaschinen in der Regel von mehreren Patienten nacheinander genutzt. Die Möglichkeit einer Übertragung von Keimen von einem Patienten zu einem anderen Patienten muss dabei unbedingt vermieden werden. Es ist Stand der Technik, dazu zwischen zwei Behandlungen eine Heißdesinfektion des Hydrauliksystems der Blutbehandlungsmaschine durchzuführen. Das Hydrauliksystem umfasst insbesondere den Behandlungsflüssigkeits-, bzw. Dialysierflüssigkeitskreislauf, und den extrakorporalen Blutkreislauf.

### Technischer Hintergrund

Eine Heißdesinfektion ist eine Keimreduktion, die in einem festgelegten Testverfahren mit geeigneten Prüfkörpern mindestens eine Keimreduktion von 10⁻⁵ erreicht. Zur Bemessung der Effektivität der Heißdesinfizierung wird das Konzept des A0-Wertes herangezogen. Er gibt die Letalität eines spezifischen Keimes bei Exposition mit einer Desinfektions-Temperatur über eine Desinfektions-Dauer an. Im Hydrauliksystem einer extrakorporalen Blutbehandlungsmaschine gibt es konstruktionsbedingt Abschnitte, in denen im Zuge der Heißdesinfizierung unterschiedlich hohe Temperaturen unterschiedlich schnell erreicht werden. Je weiter stromabwärts der Abschnitt von einer Heizeinrichtung des Hydrauliksystems entfernt liegt, umso länger dauert es einerseits, dort die erforderliche Temperatur zu erreichen. Andererseits kühlt das Fluid von einer Starttemperatur ausgehend bis dorthin ab. Die Abschnitte unterscheiden sich zudem je nach Bauform und Hersteller der Vorrichtung.

Um die o.g. geforderte Keimreduktion von 10⁻⁵ zu erreichen, werden daher von den Herstellern die erforderliche Desinfektions-Temperatur und Desinfektions-Dauer, bzw. der erforderliche A0-Wert, festgelegt und validiert, sodass sichergestellt ist, dass die Letalität des Keimes ausreichend und die Heißdesinfizierung im gesamten Hydrauliksystem wirksam ist.

Der Effektivitätsnachweis zur Heißdesinfizierung wird üblicherweise bei einer Umgebungs-/ Raumtemperatur von 23°C durchgeführt. Dass die erforderliche Desinfektions-Temperatur durch Aufheizen innerhalb einer bestimmten Zeit erreicht wird, hängt aber stark von der Umgebungstemperatur ab, der die Vorrichtung ausgesetzt ist. Bei tieferen Umgebungstemperaturen, bspw. von 15°C, insbesondere aufgrund örtlicher Gegebenheiten, kann möglicherweise der Fall eintreten, dass die Effektivität nicht mehr garantiert werden kann.

Ein fortwährendes Bestreben auf dem technischen Gebiet der extrakorporalen Blutbehandlungsmaschinen/ der Dialysemaschinen ist, den Einfluss von Umgebungsbedingungen, insbesondere der Umgebungstemperatur, auf die Laufzeit von temperaturabhängigen Maschinenprozessen kompensieren zu können und so die Laufzeit möglichst kurz halten zu können.

Die Offenlegungsschrift DE 10 2016 117 600 A1 zeigt unter diesem Aspekt ein Verfahren zur Heißdesinfektion eines Hydrauliksystems einer extrakorporalen Blutbehandlungsmaschine, bei dem das Fluid auf eine Temperatur erhitzt wird, die möglichst knapp unterhalb der druckabhängigen Siedetemperatur (Dampfdruckkurve) des Fluids liegt, sodass für den angestrebten A0-Wert eine maximal kurze Desinfektions-Dauer erzielt werden kann.

Die Druckschrift EP 3 351 275 A1 beschreibt die Abschätzung der Desinfektions-Dauer in Abhängigkeit von erfassten Umgebungsbedingungen. Als Umgebungsbedingung fließen eine Umgebungstemperatur, eine Vorlauftemperatur des Hydrauliksystems, eine Temperatur stromab des Heizgerätes und eine Temperatur am Hauptkörper der Vorrichtung ein. Ergänzend kann die Abschätzung anhand von weiteren spezifischen Parametern erfolgen, wie beispielsweise anhand einer Eingangsspannung des Heizgerätes, einer Wärmekapazität des Heizgerätes, einer Kapazität von Hydraulikleitungen, einem Druckverlust des Hydrauliksystems oder eines Montagezustandes der Vorrichtung, usw. Aus der Unterschiedlichkeit der genannten physikalischen Größen, die in die Abschätzung einfließen, resultiert eine vergleichsweise hohe Komplexität der Abschätzung.

Die Offenlegungsschrift WO 2024 056 743 A1 widmet sich der Verkürzung der Heißdesinfektion einer extrakorporalen Blutbehandlungsmaschine auf Basis von erfassten Temperaturen und von errechneten Temperaturen im Fluidkreislauf. Hierzu wird das Hydrauliksystem in mehrere Abschnitte unterteilt, in denen jeweils die Temperatur erfasst wird. Für die Abschnitte mit erfasster Temperatur wird während der Heißdesinfektion fortwährend die mit der Zeit ansteigende Letalität der dort potentiell vorhandenen Keime in Abhängigkeit der erfassten Temperatur und der Zeit berechnet. Ergänzend wird ein ortsabhängiges Temperaturprofil für das Hydrauliksystem berechnet, sodass für örtlich vorbestimmte Volumenabschnitte des Hydrauliksystems, in denen die Temperatur nicht erfasst wird, die Temperatur stattdessen berechnet wird. Für diese Volumenabschnitte (ohne Temperaturerfassung) wird während der Heißdesinfektion fortwährend die mit der Zeit ansteigende Letalität der dort potentiell vorhandenen Keime in Abhängigkeit der berechneten Temperatur und der Zeit berechnet. Mit fortschreitender Zeit wachsen in allen Abschnitten die berechneten Letalitäts-Werte (A0-Werte) an und die Heißdesinfektion wird zu einem Zeitpunkt beendet, zu dem wenigstens eine der berechneten Letalitäten oder ein Mittelwert dieser berechneten Letalitäten einen vorbestimmten Schwellenwert erreicht oder überschreitet.

### Zusammenfassung der vorliegenden Offenbarung

Die Aufgabe der vorliegenden Offenbarung ist demgegenüber, die Nachteile aus dem Stand der Technik zu vermeiden oder zumindest zu mindern und insbesondere eine extrakorporale Blutbehandlungsmaschine und ein Verfahren für einen temperaturabhängigen Maschinenprozess einer extrakorporalen Blutbehandlungsmaschine bereitzustellen, welche(s) den effizienten und sicheren Maschinenprozess der extrakorporalen Blutbehandlungsmaschine ermöglicht.

Die Aufgabe wird hinsichtlich der extrakorporalen Blutbehandlungsmaschine offenbarungsgemäß durch die Merkmale des Anspruchs 1 gelöst und hinsichtlich des Verfahrens offenbarungsgemäß durch die Merkmale des Anspruchs 11. Weiterbildungen sind in den abhängigen Ansprüchen beschrieben.

Ein Grundgedanke der vorliegenden Offenbarung sieht vor, aus Messwerten von in oder an der extrakorporalen Blutbehandlungsmaschine verbauten oder angeordneten Temperatur-Erfassungseinheiten oder -Sensoren auf eine Umgebungstemperatur zu schließen, indem ein Mittelwert der Messwerte bestimmt wird und in Abhängigkeit des bestimmten Mittelwertes eine Laufzeit eines (umgebungs-)temperaturabhängigen Maschinenprozesses der extrakorporalen Blutbehandlungsmaschine bestimmt wird. Die Laufzeit ist je nach Maschinenprozess vorzugsweise so bemessen, dass mit Ablauf der Laufzeit eine Startbedingung des Maschinenprozesses als erfüllt gilt oder dass mit Ablauf der Laufzeit eine Zielgröße des Maschinenprozesses als erreicht gilt.

Dem Grundgedanken folgend ist eine extrakorporale Blutbehandlungsmaschine, insbesondere Dialysemaschine, offenbart, die zur Durchführung wenigstens eines temperaturabhängigen Maschinenprozesses vorgesehen und ausgestaltet ist, und die zumindest aufweist:
- einen Fluidkreislauf, insbesondere einen Dialysierflüssigkeitskreislauf, sowie vorzugsweise auch einen extrakorporalen Blutkreislauf,
- eine Heizeinrichtung, die vorgesehen und ausgestaltet ist, ein Fluid des Fluidkreislaufs, insbesondere Reinstwasser, Primingflüssigkeit oder Desinfektionsflüssigkeit, zur Ausführung des Maschinenprozesses aufzuheizen,
- an Abschnitten des Fluidkreislaufs angeordnete Temperatur-Erfassungseinheiten, die vorgesehen und ausgestaltet sind, eine jeweilige Abschnitts-Temperatur des Fluids zu erfassen,
- eine hydraulische Fördereinrichtung, die vorgesehen und ausgestaltet ist, das Fluid zumindest durch den Fluidkreislauf, vorzugsweise zudem durch den extrakorporalen Blutkreislauf, wahlweise zu fördern oder nicht zu fördern, und
- eine Steuereinheit, in der eine Korrelation einer Laufzeit des wenigstens einen Maschinenprozesses in Abhängigkeit von einer Umgebungstemperatur abgelegt ist. Offenbarungsgemäß ist die Steuereinheit vorgesehen und ausgestaltet bzw. eingerichtet:
- einen Mittelwert der Abschnitts-Temperaturen zu bilden,
- in Abhängigkeit des gebildeten Mittelwertes die Umgebungstemperatur in der Korrelation zu bestimmen, und
- mittels der Korrelation in Abhängigkeit der bestimmten Umgebungstemperatur die Laufzeit des Maschinenprozesses zu bestimmen.

Auf diese Weise ist eine Blutbehandlungsmaschine bereitgestellt, die allein aufgrund ihrer "Bordmittel", das heißt mit ihren ohnehin vorhandenen Temperatur-Erfassungseinheiten, das heißt ohne auf externe Temperatursensoren angewiesen zu sein, auf die die Laufzeit beeinflussende Umgebungstemperatur schließen und daraufhin die notwendige Laufzeit des Maschinenprozesses bestimmen kann. Der temperaturabhängige Maschinenprozess der extrakorporalen Blutbehandlungsmaschine kann so effizient und sicher erfolgen.

Im Rahmen der Offenbarung ist eine Umgebung, auf sich die Umgebungstemperatur bezieht, ein Raum oder ein Raumbereich, in dem das Behandlungsgerät angeordnet ist. Alternativ ist die Umgebung ein Raumabschnitt des Behandlungsgerätes, in dem die Temperatur-Erfassungseinheiten angeordnet sind.

Entsprechend ist die Umgebungstemperatur im Rahmen der Offenbarung die Temperatur des Raumes oder Raumbereichs, in dem das Behandlungsgerät angeordnet ist, oder die Temperatur des Raumabschnitts innerhalb des Behandlungsgerätes.

Der Mittelwert der Abschnitts-Temperaturen kann in einem oder nahe einem thermischen Gleichgewicht der Temperatur-Erfassungseinheiten mit der Umgebung ermittelt werden.

Das thermische Gleichgewicht kann vorzugsweise an einem zeitlichen Verlauf der erfassten Abschnitts-Temperaturen erkannt werden. Das thermische Gleichgewicht kann daran erkannt werden, dass Gradienten der erfassten Abschnitts-Temperaturen einen Wert von null oder nahe null aufweisen und/ oder dass die erfassten Abschnitts-Temperaturen jeweils gegen einen Temperaturwert konvergieren/ konvergiert sind.

Vorzugsweise ist in der Steuereinheit ein Betrag eines Grenzgradienten abgelegt, mit dessen Unterschreiten das thermische Gleichgewicht als erreicht und erkannt gilt.

Vorzugsweise ist die Steuereinheit ausgebildet aus Verläufen der Abschnittstemperaturen einen jeweiligen Temperaturgrenzwert zu ermitteln, gegen den die Abschnitts-Temperaturen konvergieren. Vorzugswiese ist in der Steuereinheit ein Temperaturintervall abgelegt, wobei das thermische Gleichgewicht als erreicht und erkannt gilt, wenn die Abschnitts-Temperaturen in das Temperaturintervall konvergieren.

Vorzugsweise ist für mehrere temperaturabhängige Maschinenprozesse jeweils wenigstens eine Korrelation vorgesehen.

In bevorzugter Ausgestaltung ist die extrakorporale Blutbehandlungsmaschine eine Dialysemaschine, wobei der Fluidkreislauf ein Dialysierflüssigkeitskreislauf ist, der über einen Dialysierflüssigkeitseingang und einen Dialysierflüssigkeitsausgang des Dialysators durch den Dialysator verläuft. Der extrakorporale Blutkreislauf verläuft über einen Bluteingang und einen Blutausgang des Dialysators durch den Dialysator. Im Dialysator sind die Kreisläufe durch eine semipermeable Membran getrennt.

Die hydraulische Fördereinrichtung umfasst vorzugsweise eine Fluidpumpe und ein Absperrventil, wobei zum Fördern des Fluids die Fluidpumpe läuft und das Absperrventil offen ist. Das Nicht-Fördern kann durch unterschiedliche Konfigurationen erreicht werden. In einer Konfiguration läuft die Fluidpumpe nicht und das Absperrventil ist offen oder geschlossen, vorzugsweise geschlossen. In einer anderen Konfiguration läuft die Fluidpumpe, das Absperrventil ist geschlossen und die Fluidpumpe fördert im Bypass. In diesem Fall weist die Blutbehandlungsmaschine einen Bypasskreislauf zum Fluidkreislauf auf.

Vorzugsweise sind die Abschnitte des Fluidkreislaufs von stromabwärts der Heizeinrichtung bis stromaufwärts eines Fluid-Eingangs eines Hauptkörpers der Blutbehandlungsmaschine, insbesondere eines Dialysators, verteilt angeordnet.

Gemäß einer Weiterbildung ist in der Korrelation eine Prozess-Temperatur des wenigstens einen Maschinenprozesses vorbestimmt oder fest abgelegt oder eine Prozess-Temperatur des wenigstens einen Maschinenprozesses ist in Abhängigkeit der Umgebungstemperatur abgelegt. Im ersten Fall ist die Prozess-Temperatur vorbestimmt, beispielsweise für eine Heißdesinfektion. Im zweiten Fall, beispielsweise für einen Testabgleich von Temperatur-Erfassungseinheiten, kann die Steuereinheit die notwendige Prozess-Temperatur insbesondere aus dem gebildeten Mittelwert und dessen Abstand zur Umgebungstemperatur bestimmen.

Gemäß einer bevorzugten Weiterbildung ist eine erste der Temperatur-Erfassungseinheiten stromabwärts der Heizeinrichtung angeordnet. So kann über sie bei Ausführung des Maschinenprozesses die höchste Temperatur des Fluids als die Prozess-Temperatur erfasst werden.

Gemäß einer Weiterbildung hat die extrakorporale Blutbehandlungsmaschine eine Entgasungs-Einheit, über die zumindest während der Behandlung Reinstwasser entgast werden kann.

Gemäß einer Weiterbildung hat die extrakorporale Blutbehandlungsmaschine eine Mischeinheit, über die - insbesondere zur Bereitstellung frischer, individuell angepasster Dialysierflüssigkeit - dem Reinstwasser zumindest eine alkalisierende Trockensubstanz beigemischt werden kann.

Gemäß einer Weiterbildung ist die Heizeinrichtung diejenige der Entgasungs-Einheit. So kann über die erste Temperatur-Erfassungseinheit, insbesondere vor oder während einer bestimmungsgemäßen Blutbehandlung, die Temperatur des entgasten Reinstwassers erfasst werden, während in dem Maschinenprozess über sie die Prozess-Temperatur des Fluids erfasst werden kann. Die ohnehin an der Blutbehandlungsmaschine zur Kontrolle der Reinstwassertemperatur vorgesehene, erste Temperatur-Erfassungseinheit kann also vorrichtungstechnisch effizient auch für die offenbarungsgemäße Bestimmung der Laufzeit herangezogen werden.

Temperaturabhängige Maschinenprozesse sind gemäß der Offenbarung zumindest eine Heißdesinfektion des Fluidkreislaufs, vorzugsweise auch des extrakorporalen Blutkreislaufs, ein Testabgleich von Temperatur-Erfassungseinheiten bezüglich ihrer Erfassungsgenauigkeit, sowie ein Drucktest der extrakorporalen Blutbehandlungsmaschine, insbesondere des Fluidkreislaufs, des Dialysators und/ oder des extrakorporalen Blutkreislaufs.

Im Falle der Heißdesinfektion als temperaturabhängigem Maschinenprozess ist die Prozess-Temperatur eine vorzugsweise fest in der Korrelation abgelegte Heißdesinfektionstemperatur und die Laufzeit ist eine Förderdauer der hydraulischen Fördereinrichtung. Die Zielgröße der Heißdesinfektion ist vorzugsweise eine Letalität eines Keimes. Die Letalität gilt mit Ablauf der Laufzeit als erreicht.

Gemäß einer Weiterbildung ist in der Korrelation die mittels der Heißdesinfektion zu erreichende Ziel-Letalität wenigstens eines potentiell in dem Fluidkreislauf vorhandenen Keimes, vorzugsweise fest, abgelegt und die Förderdauer endet mit Erreichen, vorzugsweise mit Überschreiten, der Ziel-Letalität.

Gemäß einem Aspekt der Offenbarung kann für die Berechnung der Ziel-Letalität über die Steuereinheit der A0-Wert gemäß ISO 15883 herangezogen werden.

Gemäß einer Weiterbildung ist die Steuereinheit vorgesehen und ausgestaltet bzw. eingerichtet, die Heizeinrichtung zum Aufheizen des Fluids anzusteuern, bis die Prozess-Temperatur der Heißdesinfektion/ die Heißdesinfektionstemperatur stromabwärts der Heizeinrichtung erreicht, insbesondere von der ersten Temperatur-Erfassungseinheit erfasst, wird.

Gemäß einer Weiterbildung ist die Steuereinheit vorgesehen und ausgestaltet bzw. eingerichtet, mit Erreichen oder Erfassen der Heißdesinfektionstemperatur die Fördereinrichtung anzusteuern, das Fluid zu fördern, bis die Förderdauer, und damit die Ziel-Letalität, erreicht, vorzugsweise überschritten, ist.

Gemäß einer Weiterbildung ist die Steuereinheit vorgesehen und ausgestaltet bzw. eingerichtet, die Fördereinrichtung anzusteuern, das Fluid nicht mehr zu fördern, sobald die Laufzeit/ Förderdauer, und damit die Ziel-Letalität, erreicht, vorzugsweise überschritten ist.

Alternativ oder ergänzend weist die extrakorporale Blutbehandlungsmaschine als temperaturabhängigen Maschinenprozess einen Testabgleich von wenigstens zwei Temperatur-Erfassungseinheiten auf. Insbesondere sind diese in Strömungsrichtung (eng) benachbart, insbesondere an demselben Abschnitt oder an benachbarten Abschnitten des Fluidkreislaufs, angeordnet. Im Falle des Testabgleichs ist die Prozess-Temperatur eine Test-Temperatur und die Laufzeit ist, vorzugsweise bei gegebener Heizleistung, eine Heizdauer der Heizeinrichtung. Die Startbedingung des Testabgleichs ist vorzugsweise, dass die mit dem Aufheizen an das Fluid übertragene Wärmemenge ausreichend ist. Diese Startbedingung gilt mit Ablauf der Laufzeit als erfüllt.

Gemäß einer Weiterbildung sind an einem selben Abschnitt oder an benachbarten Abschnitten stromaufwärts eines Fluid-Eingangs eines Hauptkörpers, insbesondere eines Dialysators, der Blutbehandlungsmaschine eine fünfte und sechste der Temperatur-Erfassungseinheiten angeordnet. Über sie kann vor oder während der Blutbehandlung und im Maschinenprozess die wichtige Fluid-Eingangs-Temperatur am Hauptkörper/ Dialysator redundant erfasst werden.

Gemäß einer Weiterbildung ist in der Steuereinheit, insbesondere für den Testabgleich, eine Grenztemperatur abgelegt und die Steuereinheit ist vorgesehen und ausgestaltet bzw. eingerichtet, den bestimmten Mittelwert mit der Grenztemperatur zu vergleichen. Für Mittelwerte oberhalb der Grenztemperatur, was auf eine vergleichsweise hohe Umgebungstemperatur zurückzuführen ist, ist in der Steuereinheit die Prozess-Temperatur, insbesondere Test-Temperatur oder Heißdesinfektionstemperatur, mit einem vergleichsweise niedrigem Wert abgelegt. Für Mittelwerte unterhalb der Grenztemperatur, was auf eine vergleichsweise niedrige Umgebungstemperatur zurückzuführen ist, ist in der Steuereinheit die Prozess-Temperatur, insbesondere Test-Temperatur oder Heißdesinfektionstemperatur, mit einem vergleichsweise hohen Wert abgelegt. Auf diese Weise ist einerseits sichergestellt, dass am Ende des Fluidkreislaufs immer eine ausreichend hohe Fluid-Temperatur vorliegt, um den Maschinenprozess bestimmungsgemäß durchzuführen, und andererseits wird das Fluid nicht unnötig stark aufgeheizt, was die Energieeffizienz erhöht.

Gemäß einer Weiterbildung ist die Steuereinheit vorgesehen und ausgestaltet bzw. eingerichtet, vor Erreichen der Heizdauer die hydraulische Fördereinrichtung anzusteuern, das Fluid beim Aufheizen nicht zu fördern und mit Ablauf der Heizdauer, insbesondere mit Ablauf des Aufheizens, die hydraulische Fördereinrichtung anzusteuern, das Fluid zu fördern. Auf diese Weise wird mit Ablauf der Heizdauer ein Bolus des aufgeheizten Fluids durch den Fluidkreislauf gefördert/ geschickt.

Gemäß einer Weiterbildung ist die Steuereinheit vorgesehen und ausgestaltet, eine Differenz der von den wenigstens zwei Temperatur-Erfassungseinheiten erfassten Temperaturen zu ermitteln. Im Falle einer hinreichend kleinen ermittelten Differenz, d.h. wenn die ermittelte Differenz einen vorbestimmten Wert unterschreitet, stellt die Steuereinheit einer Ausgabeeinheit der Blutbehandlungsmaschine ein positives Testergebnis der wenigstens zwei Temperatur-Erfassungseinheiten zur Ausgabe bereit, ansonsten ein negatives Testergebnis.

Gemäß einer Weiterbildung ist die Steuereinheit vorgesehen und ausgestaltet, den Testabgleich in unterschiedlichen Temperaturbereichen des Fluids auszuführen, insbesondere in einem höheren Temperaturbereich zu Beginn oder kurz nach Beginn der Förderung des Fluids und in einem niedrigeren Temperaturbereich kurz vor Beenden oder nach Beenden der Förderung des Fluids.

Für den Testabgleich erstreckt sich der höhere Temperaturbereich insbesondere von 50°C bis 90°C, vorzugsweise von 60°C bis 80°C, besonders bevorzugt mit +/- 1°C bis 2°C um 70°C herum. Der niedrigere Temperaturbereich erstreckt sich insbesondere von 40°C bis 60°C, besonders bevorzugt mit +/- 1°C bis 2°C um 50°C herum.

Die voranstehenden Offenbarungen zur extrakorporalen Blutbehandlungsmaschine gelten ebenso für das nachfolgend beschriebene, offenbarungsgemäße Verfahren, und umgekehrt.

Ein offenbarungsgemäßes Verfahren ist für wenigstens einen temperaturabhängigen Maschinenprozess für eine oder einer extrakorporalen Blutbehandlungsmaschine, insbesondere Dialysemaschine, vorgesehen. Die extrakorporale Blutbehandlungsmaschine ist vorzugsweise gemäß wenigstens einem Aspekt der vorhergehenden Beschreibung ausgebildet und weist auf
- einen Fluidkreislauf, insbesondere einen Dialysierflüssigkeitskreislauf, sowie vorzugsweise auch einen extrakorporalen Blutkreislauf,
- eine Heizeinrichtung, die vorgesehen und ausgestaltet ist, ein Fluid des Fluidkreislaufs, insbesondere Reinstwasser, Primingflüssigkeit oder Desinfektionsflüssigkeit, zur Ausführung des Maschinenprozesses aufzuheizen,
- an Abschnitten des Fluidkreislaufs angeordnete Temperatur-Erfassungseinheiten, die vorgesehen und ausgestaltet sind, eine jeweilige Abschnitts-Temperatur des Fluids zu erfassen,
- eine hydraulische Fördereinrichtung, die vorgesehen und ausgestaltet ist, das Fluid zumindest durch den Fluidkreislauf, vorzugsweise auch durch den extrakorporalen Blutkreislauf, wahlweise zu fördern oder nicht zu fördern, und
- eine Steuereinheit, in der eine Korrelation einer Laufzeit des wenigstens einen Maschinenprozesses in Abhängigkeit von einer Umgebungstemperatur abgelegt ist. Offenbarungsgemäß weist das Verfahren folgende Schritte auf:
- Bilden eines Mittelwertes der Abschnitts-Temperaturen, über die Steuereinheit,
- Bestimmen der Umgebungstemperatur in der Korrelation in Abhängigkeit des Mittelwertes, über die Steuereinheit, und
- Bestimmen der Laufzeit des Maschinenprozesses aus der Korrelation in Abhängigkeit der Umgebungstemperatur, über die Steuereinheit.

Gemäß einer Weiterbildung des Verfahrens ist der wenigstens eine temperaturabhängige Maschinenprozess eine Heißdesinfektion, zumindest des Fluidkreislaufs.

In den Schritt Bestimmen der Laufzeit der Heißdesinfektion kann eine Aufheizzeit des Fluids und/oder eine Abkühlzeit des Fluids zumindest anteilig mit eingehen, oder sie geht/ gehen nicht mit ein. Vorzugsweise geht nur der Anteil der Aufheizzeit und/oder Abkühlzeit mit ein, in welchem die Temperatur des Fluids oberhalb einer vorbestimmten Mindesttemperatur liegt.

Gemäß einer Weiterbildung des Verfahrens sind in der Korrelation für die Heißdesinfektion zumindest abgelegt:
- als Prozess-Temperatur eine Heißdesinfektions-Temperatur, die vorzugsweise fest vorbestimmt abgelegt ist,
- eine Ziel-Letalität als A0-Wert von wenigstens einem potentiell in dem Fluidkreislauf vorhandenen Keim, die vorzugsweise fest abgelegt ist, und
- als Laufzeit eine Förderdauer der hydraulischen Fördereinrichtung in Abhängigkeit der zuvor bestimmten Umgebungs-Temperatur.

Gemäß der Offenbarung umfasst der Maschinenprozess der Heißdesinfektion Schritte:
- Ansteuern der Heizeinrichtung zum Aufheizen des Fluids, über die Steuereinheit,
- Ansteuern der Fördereinrichtung, das Fluid zu fördern, spätestens, wenn die Heißdesinfektions-Temperatur stromabwärts der Heizeinrichtung erfasst wird, und vorzugsweise frühestens, wenn die stromabwärts der Heizeinrichtung erfasste Temperatur des Fluids oberhalb der vorbestimmten Mindesttemperatur liegt, über die Steuereinheit, und
- Ansteuern der Fördereinrichtung, das Fluid nicht mehr zu fördern, wenn die Förderdauer, und damit die Ziel-Letalität, erreicht, vorzugsweise überschritten ist, über die Steuereinheit.

In alternativer oder ergänzender Weiterbildung weist das Verfahren als temperaturabhängigen Maschinenprozess einen Testabgleich von wenigstens zwei der Temperatur-Erfassungseinheiten auf, die insbesondere an demselben Abschnitt oder an benachbarten Abschnitten des Fluidkreislaufs angeordnet sind. In der Korrelation sind für den Testabgleich zumindest abgelegt:
- eine Grenztemperatur der Umgebungstemperatur, vorzugsweise fest abgelegt,
- für bestimmte Umgebungstemperaturen unterhalb der Grenztemperatur eine obere Test-Temperatur, vorzugsweise fest abgelegt,
- für bestimmte Umgebungstemperaturen oberhalb der Grenztemperatur eine untere Test-Temperatur, vorzugsweise fest abgelegt, und
- als Laufzeit eine Heizdauer der Heizeinrichtung.

Gemäß der Offenbarung umfasst der Maschinenprozess des Testabgleichs Schritte:
- Vergleichen der bestimmten Umgebungstemperatur mit der Grenztemperatur, über die Steuereinheit,
- Bestimmen der oberen Test-Temperatur, falls die bestimmte Umgebungstemperatur kleiner als die Grenztemperatur ist, über die Steuereinheit,
- Bestimmen der unteren Test-Temperatur, falls die bestimmte Umgebungstemperatur größer als die Grenztemperatur ist, über die Steuereinheit, und
- Bestimmen einer Differenz der bestimmten Test-Temperatur zur bestimmten Umgebungstemperatur, über die Steuereinheit.

Diese Differenz geht dann in den offenbarungsgemäßen Schritt Bestimmen der (Laufzeit) Heizdauer des (Maschinenprozesses) Testabgleichs mittels der Korrelation in Abhängigkeit der bestimmten Umgebungstemperatur ein.

Ist eine Heizleistung vorgegeben oder fest, so ist die Heizdauer umso länger, je größer die Differenz ist.

Gemäß der Offenbarung umfasst der Maschinenprozess des Testabgleichs weitere Schritte:
- Ansteuern der Heizeinrichtung, das Fluid für die bestimmte Heizdauer aufzuheizen, über die Steuereinheit,
- Ansteuern der hydraulischen Fördereinrichtung, das Fluid vor Ablauf der Heizdauer nicht zu fördern, über die Steuereinheit,
- Ansteuern der hydraulischen Fördereinrichtung, das Fluid mit Ablauf der Heizdauer zu fördern, über die Steuereinheit,
- Ermitteln einer Differenz der von den wenigstens zwei Temperatur-Erfassungseinheiten erfassten Temperaturen, über die Steuereinheit,
- Bereitstellen eines positiven Testergebnisses an eine Ausgabeeinheit der Blutbehandlungsmaschine im Falle einer hinreichend kleinen Differenz, d.h. wenn die ermittelte Differenz einen vorbestimmten Wert unterschreitet, und ansonsten Bereitstellen eines negativen Testergebnisses an die Ausgabeeinheit.

Zumindest der Schritt Ermitteln der Differenz und die Schritte Bereitstellen des Testergebnisses werden vorzugsweise in mehreren Temperaturbereichen des Fluids und/ oder Zeiträumen des Testabgleichs wiederholt, um die Genauigkeit der Temperatur-Erfassungseinheiten in mehreren Temperaturbereichen zu testen.

Bevorzugt erfolgt der Testabgleich in dem höheren Temperaturbereich in einem Zeitraum nach Beginn der Förderung des Fluids, sowie in dem niedrigeren Temperaturbereich in einem Zeitraum nach Beenden der Förderung des Fluids.

Gemäß einer Weiterbildung erfolgt das Ermitteln des Mittelwertes gewichtet, vorzugsweise in Abhängigkeit einer Eigenschaft des jeweiligen Abschnitts, insbesondere eines Volumens oder einer Wärmekapazität.

Das offenbarungsgemäße Verfahren wird ausgeführt, wenn ein Patient nicht an die extrakorporale Blutbehandlungsmaschine, das heißt nicht an den extrakorporalen Kreislauf angeschlossen ist, so dass das offenbarungsgemäße Verfahren kein Verfahren zur chirurgischen oder therapeutischen Behandlung des menschlichen oder tierischen Körpers und auch kein Diagnostizierverfahren, das am menschlichen oder tierischen Körper vorgenommen wird, ist.

Das heißt zum einen, dass das Verfahren und der/ die temperaturabhängige(n) Maschinenprozess(e) abgeschlossen ist/ sind, bevor eine extrakorporale Blutbehandlung (Therapie) beginnt, und zum anderen, dass das Verfahren und der/ die temperaturabhängige(n) Maschinenprozess(e) erst beginnen, nachdem eine extrakorporale Blutbehandlung (Therapie) abgeschlossen ist.

Vorzugsweise läuft das Verfahren und der/ die temperaturabhängige(n) Maschinenprozess(e) am Ende einer Ruhephase eines Behandlungsbetriebes, in dem das Behandlungsgerät ausgeschaltet ist. Die Ruhephase ist beispielsweise ein nächtlicher Zeitraum, beispielsweise ein Zeitraum zwischen 22 Uhr abends und 6 Uhr morgens.

Die offenbarungsgemäße Vorrichtung kann grundsätzlich auch eingerichtet sein, den Maschinenprozess bei angeschlossenem Patienten auszuführen, bevorzugt ist jedoch, dass der Maschinenprozess bei nicht angeschlossenem Patienten ausgeführt wird.

Gemäß der Offenbarung umfasst ein Computerprogramm Befehle, die bei einer Ausführung durch einen Computer diesen veranlassen die Verfahrensschritte des Verfahrens nach wenigstens einem Aspekt der vorangegangenen Beschreibung auszuführen.

Gemäß der Offenbarung umfasst ein computerlesbares Speichermedium Befehle, die bei einer Ausführung durch einen Computer diesen veranlassen die Verfahrensschritte des Verfahrens nach wenigstens einem Aspekt der vorangegangenen Beschreibung auszuführen.

### Kurzbeschreibung der Figuren

Die Offenbarung wird nachfolgend anhand bevorzugter Ausführungsformen mit Hilfe von Figuren näher erläutert. Es zeigen:
- Fig. 1: eine schematische Ansicht einer offenbarungsgemäßen, extrakorporalen Blutbehandlungsmaschine, gemäß einer Ausführungsform;
- Fig. 2: ein Flussdiagramm eines Verfahrens für einen ersten temperaturabhängigen Maschinenprozess (Heißdesinfektion) der extrakorporalen Blutbehandlungsmaschine gemäß Figur 1, gemäß einer bevorzugten Ausführungsform; und
- Fig. 3: ein Flussdiagramm eines Verfahrens für einen zweiten temperaturabhängigen Maschinenprozess (Testabgleich) der extrakorporalen Blutbehandlungsmaschine gemäß Figur 1, gemäß einer bevorzugten Ausführungsform.

Die Figuren sind schematischer Natur und sollen nur dem Verständnis der Offenbarung dienen. Gleiche Elemente sind mit denselben Bezugszeichen versehen. Merkmale verschiedener Ausführungsformen können untereinander ausgetauscht werden.

### Detaillierte Beschreibung bevorzugter Ausführungsformen

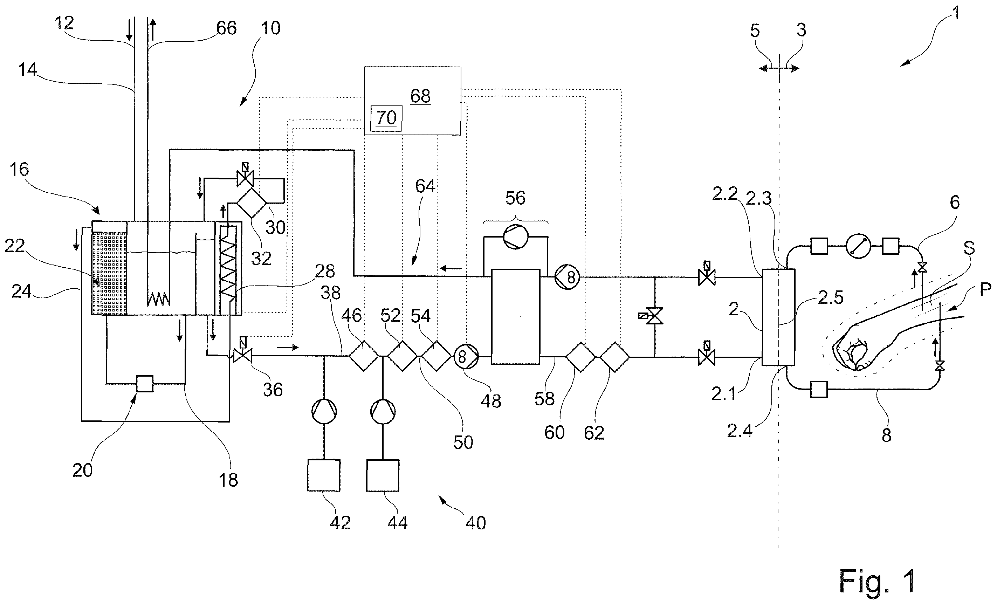

Figur 1 zeigt in einer schematischen Ansicht eine extrakorporale Blutbehandlungsmaschine 1 in Form einer Dialysemaschine gemäß einer bevorzugten Ausführungsform der vorliegenden Offenbarung. Die Blutbehandlungsmaschine 1 weist als zentrale Komponente einen Dialysator 2 mit einerseits einem dialysierflüssigkeitsseitigen Dialysierflüssigkeitseingang 2.1 und Dialysatausgang 2.2 und andererseits einem blutseitigen Bluteingang 2.3 und Blutausgang 2.4 eines extrakorporalen Blutkreislaufs 3 auf. Innerhalb ist der Dialysator 2 mittels Hohlfasern einer semipermeablen Membran 2.5 in eine Dialysierflüssigkeitsseite eines Dialysierflüssigkeitskreislaufs oder Fluidkreislaufs 5 und eine Blutseite eines extrakorporalen Blutreislaufs 3 der Blutbehandlungsmaschine 1 aufgeteilt.

Der extrakorporale Blutkreislauf 3 hat einen arteriellen Schlauchabschnitt 6, um dem Patienten P Blut über einen Shunt S zu entnehmen und über den Bluteingang 2.3 dem Dialysator 2 zuzuführen. Nachdem im extrakorporalen Blutkreislauf 3 das Blut des Patienten P durch die Blutseite des Dialysators 2 geleitet wurde, wird es an dessen Blutausgang 2.4 entnommen und über einen venösen Schlauchabschnitt 8 dem Shunt S zugeführt. In dem Dialysator 2 wird das Blut im Gegenstromverfahren zur Dialysierflüssigkeit/ zum Fluid geführt und von harnpflichtigen Bestandteilen und überschüssigem Wasser befreit und hiernach gereinigt dem Patienten P zurückgegeben/ zurückgeführt.

Der Fluidkreislauf 5 hat zur Bereitstellung von entgastem oder teilentgastem Reinstwasser eine Entgasungseinheit 10, deren Eingang 12 einerseits mit einem Versorgungsanschluss einer Umkehrosmose-Anlage (nicht dargestellt) verbunden ist und andererseits über einen Reinstwasserzulauf 14 in einen Entgasungstank 16 mündet. Bodenseitig am Entgasungstank 16 setzt ein Entgasungskanal 18 an, der über eine Drosseleinrichtung 20 fluidisch mit einer Entgasungskammer 22 verbunden ist. Vom Scheitel der Entgasungskammer 22 führt ein Verbindungskanal 24 in eine Heizeinrichtung 28 des Entgasungstanks 16.

Stromabwärts der Heizeinrichtung 28 ist an einem ersten Abschnitt 30 des Fluidkreislaufs 5 eine erste Temperatur-Erfassungseinheit 32 angeordnet, über die eine Fluid- oder erste Abschnitts-Temperatur T1 des Fluidkreislaufs 5 erfasst wird. Die erste Abschnitts-Temperatur T1 wird im Folgenden gleichbedeutend als (zu erreichende) Prozess-Temperatur Tp eines Maschinenprozesses der offenbarungsgemäßen, extrakorporalen Blutbehandlungsmaschine 1 bezeichnet.

Eine Beruhigungskammer des Entgasungstanks 16 ist bodenseitig über ein Sperrventil 36 mit einem zweiten Abschnitt 38 des Fluidkreislaufs 5, der einer Mischeinheit 40 zugeordnet ist, verbunden. Über die Mischeinheit 40 kann dem zulaufenden, zumindest teilentgasten Reinstwasser, zur individuellen Herstellung frischer Dialysierflüssigkeit ein basisches Konzentrat und ein saures Konzentrat zu-dosiert/ beigemischt werden. Dementsprechend hat die Mischeinheit 40 eine erste und zweite Quelle 42, 44 für basisches und saures Konzentrat.

Der zweite Abschnitt 38 des Fluidkreislaufs 5 erstreckt sich zwischen Einmündungen der Quellen 42, 44 für basisches und saures Konzentrat. Am zweiten Abschnitt 38 ist eine zweite Temperatur-Erfassungseinheit 46 des Fluidkreislaufs 5 zur Erfassung einer zweiten Abschnitts-Temperatur T2 vorgesehen.

Von stromabwärts der Einmündung der Quelle 44 für saures Konzentrat bis stromaufwärts einer Fluid-Pumpe 48 erstreckt sich ein dritter Abschnitt 50 des Fluidkreislaufs 5.

An dem dritten Abschnitt 50 sind eine dritte und eine vierte Temperatur-Erfassungseinheit 52, 54 zur Erfassung einer dritten und vierten Abschnitts-Temperatur T3, T4 des Fluidkreislaufs 5 angeordnet. Die dritte Temperatur-Erfassungseinheit 52 ist dabei eine Haupt-Erfassungseinheit und die vierte Temperatur-Erfassungseinheit 54 eine Redundanz- oder Referenz-Erfassungseinheit.

Stromabwärts des dritten Abschnitts 50 und einer Bilanzierungskammer 56 des Fluidkreislaufs 5 schließt ein vierter Abschnitt 58 des Fluidkreislauf 5 an, an dem eine fünfte und eine sechste Temperatur-Erfassungseinheit 60, 62 angeordnet sind. Sie sind vorgesehen, eine fünfte und sechste Abschnitts-Temperatur T5, T6 am vierten Abschnitt 58 des Fluidkreislaufs 5 zu erfassen, die eine Fluid-Eingangs-Temperatur am Dialysierflüssigkeitseingang 2.1 des Dialysators 2 repräsentieren. Die fünfte Temperatur-Erfassungseinheit 60 ist dabei eine Haupt-Erfassungseinheit und die sechste Temperatur-Erfassungseinheit 62 eine Redundanz- oder Referenz-Erfassungseinheit.

Die vier genannten Abschnitte 30, 38, 50, 58 des Fluidkreislaufs 5 erstrecken sich von stromab der Heizeinrichtung 28 bis zum Dialysierflüssigkeitseingang 2.1 des Dialysators 2 und bilden so einen Abschnitt eines Zulaufs des Fluidkreislaufs 5 aus.

Vom Dialysierflüssigkeitsausgang 2.2 des Dialysators 2 ausgehend erstreckt sich ein Rücklauf 64 des Fluidkreislaufs 5 über die Bilanzierungskammer 56, über eine Wärmerückgewinnung im Entgasungstank 16, hin zu einem Entsorgungsausgang 66 für verbrauchte Dialysierflüssigkeit oder Dialysat.

Die extrakorporale Blutbehandlungsmaschine 1 hat eine Steuereinheit 68, in der eine Korrelation 70 für wenigstens einen temperaturabhängigen Maschinenprozess abgelegt ist. Ein derartiger Maschinenprozess ist offenbarungsgemäß insbesondere:
- eine Heißdesinfektion des Fluidkreislaufs und optional ergänzend des extrakorporalen Blutkreislaufs; oder
- ein Testabgleich zumindest der am gleichen Abschnitt 50, bzw. 58 angeordneten Temperatur-Erfassungseinheiten 52, 54, bzw. 60, 62.

Die Heißdesinfektion ist eine Keimreduktion, die in einem festgelegten Testverfahren mit geeigneten Prüfkörpern mindestens eine Keimreduktion von 10⁻⁵ erreichen sollte. Um diese Keimreduktion sicherzustellen, wird von Herstellern von Dialysemaschinen die Zeit zur Durchführung der Heißdesinfektion vorbestimmt und die erforderliche Heißdesinfektions- oder Prozess-Temperatur, bzw. der A0-Wert, wird festgelegt und validiert. Ein Problem hierbei ist, dass es stark von der Umgebungstemperatur abhängig ist, ob die erforderliche Heißdesinfektions- oder Prozess-Temperatur in der gegebenen Zeit erreicht wird, oder nicht. Der Effektivitätsnachweis der Heißdesinfektion wird üblicherweise bei Raumtemperatur (ca. 23°C) durchgeführt. Bei niedrigeren Umgebungstemperaturen, bspw. bei 15°C, kann möglicherweise die Effektivität nicht garantiert werden. Es ist also vorteilhaft, die Umgebungstemperatur zu kennen, um so ggf. die Laufzeit der Heißdesinfektion anzupassen und auf diese Weise die Effektivität auch bei niedrigeren Temperaturen sicherzustellen. Hier setzt für den Maschinenprozess Heißdesinfektion die Offenbarung wie folgt an:
Über die Steuereinheit 68 wird ein Mittelwert Tx der von den maschineninternen Temperatur-Erfassungseinheiten 32, 46, 52, 54, 60, 62 erfassten Abschnitts-Temperaturen T1 bis T6 des Fluidkreislaufs 5 gebildet. Dieser Mittelwert Tx repräsentiert in der Korrelation 70 offenbarungsgemäß die bestimmte Umgebungstemperatur Tu. Aus der Korrelation 70 wird dann in Abhängigkeit der bestimmten Umgebungstemperatur Tu die Laufzeit tp für kältere Umgebungstemperaturen Tu verlängert und für wärmere Umgebungstemperaturen Tu verkürzt bestimmt.

Zur Ausführung der Maschinenprozesse ist die Steuereinheit 68 gemäß der Offenbarung mit folgenden Komponenten signalverbunden:
- mit der Heizeinrichtung 28, die vorgesehen und ausgestaltet ist, in Abhängigkeit der Ansteuerung durch die Steuereinheit 68 das Fluid des Fluidkreislaufs 5, insbesondere das Reinstwasser, zur Ausführung des Maschinenprozesses aufzuheizen;
- mit den Temperatur-Erfassungseinheiten 32, 46, 52, 54, 60, 62, die vorgesehen und ausgestaltet sind, die Abschnitts-Temperaturen T1, T2, T3, T4, T5, T6 des Fluids im Fluidkreislauf zu erfassen und der Steuereinheit 68 bereitzustellen; und
- mit einer hydraulischen Fördereinrichtung, die zumindest das Absperrventil 36 und die Fluidpumpe 48 umfasst, und die vorgesehen und ausgestaltet ist, in Abhängigkeit der Ansteuerung durch die Steuereinheit 68 das Fluid wahlweise zu fördern oder nicht zu fördern.

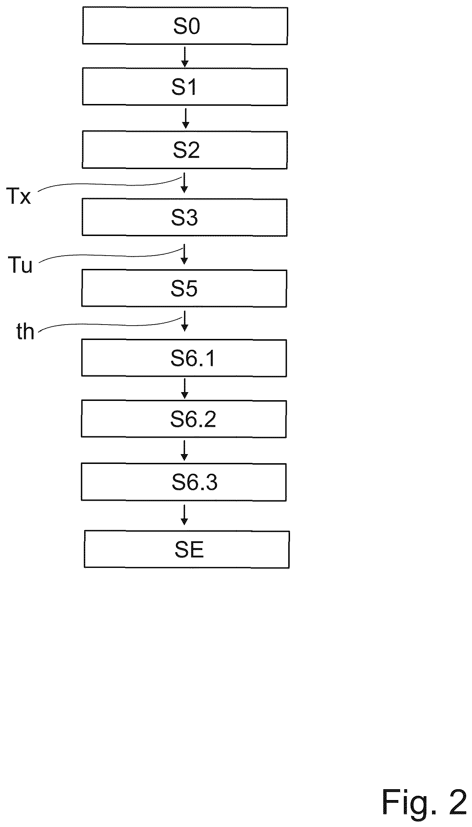

Figur 2 zeigt für den speziellen temperaturabhängigen Maschinenprozess einer Heißdesinfektion H der extrakorporalen Blutbehandlungsmaschine 1 ein Flussdiagramm eines offenbarungemäßen Verfahrens gemäß einer bevorzugten Ausführungsform. Die Beschreibung des Verfahrens gemäß Figur 2 erfolgt unter Bezug auf Figur 1.

Zu Beginn des Verfahrens gemäß Figur 2 erfolgt ein Schritt Starten oder Initialisieren **S0** des Maschinenprozesses Heißdesinfektion H, insbesondere manuell über einen Benutzer oder automatisiert/ zeitgesteuert durch die Steuereinheit 68.

Es folgt ein Schritt Erfassen **S1** der Abschnitt-Temperaturen T1, T2, T3, T4, T5, T6 über die Temperatur-Erfassungseinheiten 32, 46, 52, 54, 60, 62, die die erfassten Werte an die signalverbundene Steuereinheit 68 übermitteln. Die Erfassung erfolgt zumindest initial und kann ergänzend kontinuierlich erfolgen oder periodisch wiederholt werden.

Darauf basierend erfolgt ein Schritt Bilden **S2** des Mittelwertes Tx der erfassten Abschnitts-Temperaturen T1, T2, T3, T4, T5, T6 über die Steuereinheit 68. Der Mittelwert Tx kann dabei offenbarungsgemäß für eine Anzahl von n Temperatur-Erfassungseinheiten durch folgende in der Steuereinheit 68 aufrufbar abgelegte Berechnungsvorschrift gebildet werden: $Tx = \left(T1+T2+T3+T4+…+Tn\right) / n$

Alternativ oder ergänzend hierzu kann beispielsweise eine gewichtete Mittelung erfolgen.

Der Mittelwert Tx kann in einem oder nahe einem thermischen Gleichgewicht der Temperatur-Erfassungseinheiten 32, 46, 52, 54, 60, 62 mit der Umgebung ermittelt werden.

Das thermische Gleichgewicht kann vorzugsweise an einem zeitlichen Verlauf der erfassten Abschnitts-Temperaturen T1, T2, T3, T4, T5, T6 erkannt werden. Das thermische Gleichgewicht kann daran erkannt werden, dass Gradienten der erfassten Abschnitts-Temperaturen T1, T2, T3, T4, T5, T6 einen Wert von null oder nahe null aufweisen und/ oder dass die erfassten Abschnitts-Temperaturen T1, T2, T3, T4, T5, T6 jeweils gegen einen Temperaturwert konvergieren/ konvergiert sind.

Vorzugsweise ist in der Steuereinheit 68 ein Betrag eines Grenzgradienten abgelegt, mit dessen Unterschreiten das thermische Gleichgewicht als erreicht und erkannt gilt.

Vorzugsweise ist die Steuereinheit 68 ausgebildet, aus Verläufen der Abschnittstemperaturen T1, T2, T3, T4, T5, T6 einen jeweiligen Temperaturgrenzwert zu ermitteln, gegen den die Abschnitts-Temperaturen T1, T2, T3, T4, T5, T6 konvergieren. Vorzugswiese ist in der Steuereinheit 68 ein Temperaturintervall abgelegt, wobei das thermische Gleichgewicht als erreicht und erkannt gilt, wenn die Abschnitts-Temperaturen T1, T2, T3, T4, T5, T6 in das Temperaturintervall konvergieren.

Im Anschluss ruft die Steuereinheit 68 die Korrelation 70 auf, in der die Laufzeit th der Heißdesinfektion H in Abhängigkeit der Umgebungstemperatur Tu abgelegt ist und es folgt ein Schritt Bestimmen **S3** der Umgebungstemperatur Tu in Abhängigkeit des gebildeten Mittelwertes Tx in der Korrelation 70, über die Steuereinheit 68.

Man kann in anderen Worten sagen, der Mittelwert Tx wird in der Korrelation 70 als Wert der Umgebungstemperatur Tu gesetzt.

Dieses Setzen des Mittelwertes Tx als Umgebungstemperatur Tu setzt voraus, dass das Fluid innerhalb der Dialysemaschine 1 nach deren Betrieb bereits abgekühlt ist, sodass sich die Abschnitts-Temperaturen T1, T2, T3, T4, T5, T6 thermisch ausreichend an die Umgebungstemperatur angeglichen haben.

Es folgt der Schritt Bestimmen **S5** der Laufzeit th der Heißdesinfektion H in Abhängigkeit der Umgebungstemperatur Tu/ des Mittelwertes Tx, über die Steuereinheit 68.

Die Korrelation 70 kann als Rechenvorschrift oder als Look-up-Tabelle abgelegt sein. Gemäß der Offenbarung sind für die Heißdesinfektion H in der Korrelation 70 abgelegt: als Prozess-Temperatur eine Heißdesinfektions-Temperatur Th, vorzugsweise ist diese als fester Wert abgelegt, eine Ziel-Letalität A0 wenigstens eines potentiell in dem Fluidkreislauf 5 vorhandenen Keimes, vorzugsweise ist auch diese fest abgelegt, und als Laufzeit eine Förderdauer th der hydraulischen Fördereinrichtung 36, 48.

Eine mögliche Implementierung dieser Korrelation 70 für die Heißdesinfektion H ist beispielsweise eine (unten dargestellte) Tabelle, aus der anschaulich hervorgeht, dass mit sinkender Umgebungstemperatur Tu, das heißt sinkendem Mittelwert Tx, die Laufzeit th der Heißdesinfektion H ansteigen muss, um bei vorgegebener Heißdesinfektionstemperatur Th die Ziel-Letalität A0 von 1200s zu erreichen.

In der dargestellten Korrelation 70 handelt es sich bevorzugt um eine Korrelation zur zitrothermische Desinfektion und die Ziel-Letalität A0 des potentiell im Fluidkreislauf 5 auftretenden Keims ist mit 1200s vorgegeben.

| | Tu / Tx [°C] | A0 [s] | th [min] |
|---|---|---|---|
| H | 30 | 1200 | 22 |
| H | 25 | 1200 | 23 |
| H | 20 | 1200 | 26 |
| H | 15 | 1200 | 30 |

Nachdem die Laufzeit bekannt ist, folgen gemäß Figur 2 die Schritte:
- Ansteuern **S6.1** der Heizeinrichtung 28 zum Aufheizen des Fluids, über die Steuereinheit 68,
- Ansteuern **S6.2** der Fördereinrichtung 36, 48, das Fluid zu fördern, sobald die Heißdesinfektions-Temperatur Th stromabwärts der Heizeinrichtung 28 an der ersten Temperatur-Erfassungseinheit 32 erfasst wird, hierfür wird insbesondere die Fluidpumpe 48 gestartet und das Absperrventil 36 geöffnet, und
- Ansteuern **S6.3** der Fördereinrichtung 36, 48, das Fluid nicht mehr zu fördern, wenn die Förderdauer th, und damit die Ziel-Letalität A0, erreicht, vorzugsweise überschritten, ist. Hierfür wird insbesondere die Fluidpumpe 48 gestoppt und das Absperrventil 36 geschlossen.

Es folgt der Schritt Beenden **SE** des Maschinenprozesses, das heißt, der Heißdesinfektion H.

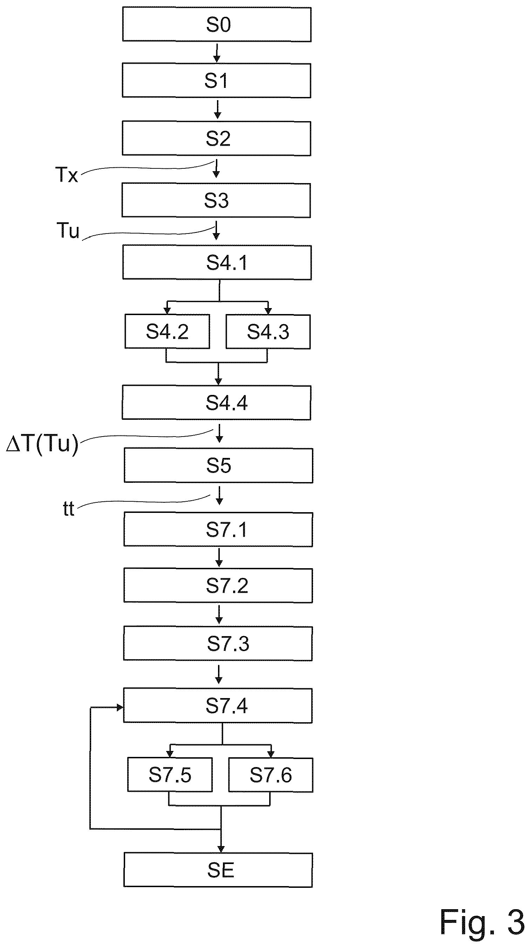

Figur 3 zeigt für den speziellen temperaturabhängigen Maschinenprozess eines Testabgleichs T der Temperatur-Erfassungseinheiten 60, 62 nahe des Dialysierflüssigkeitseingangs 2.1 ein Flussdiagramm eines offenbarungemäßen Verfahrens gemäß einer bevorzugten Ausführungsform. Die Beschreibung des Verfahrens gemäß Figur 3 erfolgt unter Bezug auf Figur 1.

Zunächst erfolgt der Schritt Starten oder Initialisieren **S0** des Maschinenprozesses Testabgleich T, insbesondere manuell über einen Benutzer oder automatisiert/ zeitgesteuert durch die Steuereinheit 68.

Wie bei dem Maschinenprozess Heißdesinfektion H folgen die Schritte Erfassen **S1** der Abschnitt-Temperaturen und Bilden **S2** des Mittelwertes Tx der erfassten Abschnitts-Temperaturen T1, T2, T3, T4, T5, T6, sowie Bestimmen **S3** der Umgebungstemperatur Tu in Abhängigkeit des Mittelwertes Tx in einer dem Testabgleich zugeordneten Korrelation 70, über die Steuereinheit 68. Man kann in anderen Worten sagen, der Mittelwert Tx wird in der Korrelation 70 als Wert der Umgebungstemperatur Tu gesetzt.

Für den Testabgleich T sind in der Korrelation 70 zumindest abgelegt:
- eine Grenztemperatur Tg der Umgebungstemperatur Tu, um unterschiedliche Testabläufe bei warmer und bei kalter Umgebungstemperatur Tu durchführen zu können,
- für Umgebungstemperaturen Tu unterhalb der Grenztemperatur Tg eine, vorzugsweise feste, obere Test-Temperatur Tto,
- für Umgebungstemperaturen Tu oberhalb der Grenztemperatur (Tg) eine, vorzugsweise feste, untere Test-Temperatur Ttu,
- als Laufzeit tp eine Heizdauer tt der Heizeinrichtung 28, und
- vorzugsweise eine feste Heizleistung der Heizeinrichtung 28.

Bevor die Heizdauer tt ermittelt wird, wird zunächst geklärt, ob eine "kalte" oder eine "warme" Testumgebung vorliegt. Hierzu weist das Verfahren gemäß der Offenbarung Schritte auf:
- Vergleichen **S4.1** der bestimmten Umgebungstemperatur Tu/ bzw. des Mittelwertes Tx, mit der Grenztemperatur Tg,
- Bestimmen/ Aufrufen **S4.2** der oberen Test-Temperatur Tto, falls die bestimmte Umgebungstemperatur Tu kleiner als die Grenztemperatur Tg ist,
- und Bestimmen/ Aufrufen **S4.3** der unteren Test-Temperatur Ttu, falls die bestimmte Umgebungstemperatur Tu größer als die Grenztemperatur Tg ist, jeweils über die Steuereinheit 68.

Da die Heizleistung im gezeigten Ausführungsbeispiel vorbestimmt und fest ist, erfolgt zur Bestimmung der Heizdauer zunächst ein Schritt Bestimmen **S4.4** einer Temperaturdifferenz ΔT aus der bestimmten Test-Temperatur Tto, bzw. Ttu und der Umgebungstemperatur Tu, bzw. dem bestimmten Mittelwert Tx, über die Steuereinheit 68.

Die bestimmte Temperaturdifferenz ΔT geht dann in den Schritt Bestimmen **S5** der Heizdauer tt des Testabgleichs T in Abhängigkeit der Umgebungstemperatur Tu ein.

Anschließend erfolgen die Schritte Ansteuern **S7.1** der Heizeinrichtung 28, um das Fluid für die bestimmte Heizdauer tt aufzuheizen, Ansteuern **S7.2** der hydraulischen Fördereinrichtung 36, 48, das Fluid vor Ablauf der Heizdauer tt nicht zu fördern, Ansteuern **S7.3** der hydraulischen Fördereinrichtung 36, 48, das Fluid mit Ablauf der Heizdauer tt zu fördern, Ermitteln **S7.4** einer Differenz Td der von den zwei Temperatur-Erfassungseinheiten 60, 62 erfassten Temperaturen T5, T6, und Bereitstellen **S7.5** eines positiven Testergebnisses an eine Ausgabeeinheit der Blutbehandlungsmaschine 1 im Falle einer hinreichend kleinen Differenz Td, wenn also die ermittelte Differenz Td einen vorbestimmten Wert unterschreitet, und ansonsten, wenn also die ermittelte Differenz Td einen vorbestimmten Wert überschreitet, Bereitstellen **S7.6** eines negativen Testergebnisses an die Ausgabeeinheit.

Die Schritte Ermitteln S7.4 der Differenz Td und die Schritte Bereitstellen S7.5, S7.6 des Testergebnisses werden vorzugsweise in mehreren Temperaturbereichen Tb und/ oder Zeiträumen wiederholt, bevorzugt in einem höheren Temperaturbereich in einem Zeitraum nach Beginn der Förderung des Fluids, sowie in einem niedrigeren Temperaturbereich in einem Zeitraum nach Beenden der Förderung des Fluid.

### Bezugszeichenliste

- 1: Extrakorporale Blutbehandlungsmaschine
- 2: Dialysator
- 2.1: Dialysierflüssigkeitseingang
- 2.2: Dialysatausgang
- 2.3: Bluteingang
- 2.4: Blutausgang
- 2.5: semipermeable Membran
- 3: extrakorporaler Blutkreislauf
- 5: Dialysierflüssigkeitskreislauf/ Fluidkreislauf
- 6: arterieller Abschnitt
- 8: venöser Abschnitt
- 10: Entgasungseinheit
- 12: Eingang Entgasungseinheit
- 14: Reinstwasserzulauf
- 16: Entgasungstank
- 18: Entgasungskanal
- 20: Drosseleinrichtung
- 22: Entgasungskammer
- 24: Verbindungskanal
- 28: Heizeinrichtung
- 30: erster Abschnitt
- 32: erste Temperatur-Erfassungseinheit
- 36: Sperrventil
- 38: zweiter Abschnitt
- 40: Mischeinheit
- 42: alkalisierende Quelle
- 44: säuernde Quelle
- 46: zweite Temperatur-Erfassungseinheit
- 50: dritter Abschnitt
- 52: dritte Temperatur-Erfassungseinheit
- 54: vierte Temperatur-Erfassungseinheit
- 56: Bilanzierungskammer
- 58: vierter Abschnitt
- 60: fünfte Temperatur-Erfassungseinheit
- 62: sechste Temperatur-Erfassungseinheit
- 64: Rücklauf
- 66: Entsorgungsausgang
- 68: Steuereinheit
- 70: Korrelation

- S0: Start Maschinenprozess
- S1: Erfassen Temperaturen
- S2: Bilden Mittelwert
- S3: Bestimmen Umgebungstemperatur
- S4.1: Vergleichen Grenztemperatur
- S4.2, S4.3: Bestimmen Testtemperatur
- S4.4: Bestimmen Temperaturdifferenz
- S5: Bestimmen Laufzeit
- S6.1: Ansteuern Heizeinrichtung
- S6.2, S6.3: Ansteuern Fördereinrichtung
- S7.1: Ansteuern Heizeinrichtung
- S7.2, S7.3: Ansteuern Fördereinrichtung
- S7.4: Ermitteln Differenz
- S7.5, S7.6: Bereitstellen Testergebnis
- SE: Ende Maschinenprozess

- P: Patient
- T1, T2, T3, T4, T5, T6: erste bis sechste Abschnitts-Temperatur
- Tx: Mittelwert Abschnitts-Temperaturen
- Tu: Umgebungstemperatur
- H: Maschinenprozess Heißdesinfektion
- T: Maschinenprozess Testabgleich
- th: Laufzeit Heißdesinfektion
- tt: Laufzeit Testabgleich
- Δt: Temperaturdifferenz

## Patentansprüche

1. Extrakorporale Blutbehandlungsmaschine (1), insbesondere Dialysemaschine (1), die zur Durchführung wenigstens eines temperaturabhängigen Maschinenprozesses (H, T) vorgesehen und ausgestaltet ist, zumindest aufweisend:
- einen Fluidkreislauf (5), insbesondere einen Dialysierflüssigkeitskreislauf,
- eine Heizeinrichtung (28), die vorgesehen und ausgestaltet ist, ein Fluid des Fluidkreislaufs (5), insbesondere Reinstwasser, Primingflüssigkeit oder Desinfektionsflüssigkeit, zur Ausführung des Maschinenprozesses (H, T) aufzuheizen,
- an Abschnitten (30, 38, 50, 58) des Fluidkreislaufs (5) angeordnete Temperatur-Erfassungseinheiten (32, 46, 52, 54, 60, 62), die vorgesehen und ausgestaltet sind, eine jeweilige Abschnitts-Temperatur (T1, T2, T3, T4, T5, T6) des Fluids zu erfassen,
- eine hydraulische Fördereinrichtung (36, 48), die vorgesehen und ausgestaltet ist, das Fluid wahlweise zu fördern oder nicht zu fördern, und
- eine Steuereinheit (68), in der eine Korrelation (70) einer Laufzeit (tp) des wenigstens einen Maschinenprozesses (H, T) in Abhängigkeit von einer Umgebungstemperatur (Tu) abgelegt ist, **dadurch gekennzeichnet, dass** die Steuereinheit (68) vorgesehen und ausgestaltet ist,
- einen Mittelwert (Tx) der erfassten Abschnitts-Temperaturen (T1, T2, T3, T4, T5, T6) zu bilden,
- in Abhängigkeit des gebildeten Mittelwertes (Tx) in der Korrelation (70) die Umgebungstemperatur (Tu) zu bestimmen, und
- mittels der Korrelation (70) in Abhängigkeit der Umgebungstemperatur (Tu) eine Laufzeit (tp) des Maschinenprozesses (H, T) zu bestimmen.

2. Extrakorporale Blutbehandlungsmaschine (1) nach Anspruch 1, **dadurch gekennzeichnet, dass** in der Korrelation (70) eine Prozess-Temperatur (Tp) des wenigstens einen Maschinenprozesses (H) fest abgelegt ist oder eine Prozess-Temperatur (Tp) des wenigstens einen Maschinenprozesses (T) in Abhängigkeit der Umgebungstemperatur (Tu) abgelegt ist.

3. Extrakorporale Blutbehandlungsmaschine (1) nach Anspruch 2, **dadurch gekennzeichnet, dass** eine erste (32) der Temperatur-Erfassungseinheiten (32, 46, 52, 54, 60, 62) stromabwärts der Heizeinrichtung (28) angeordnet ist, um die Prozess-Temperatur (Tp) des Fluids zu erfassen.

4. Extrakorporale Blutbehandlungsmaschine (1) nach Anspruch 2 oder 3, **dadurch gekennzeichnet, dass** der wenigstens eine, temperaturabhängige Maschinenprozess eine Heißdesinfektion (H) zumindest des Fluidkreislaufs (5) ist, die Prozess-Temperatur (Tp) eine Heißdesinfektions-Temperatur (Th) ist, die vorzugsweise fest abgelegt ist, und die Laufzeit (tp) eine Förderdauer (th) der hydraulischen Fördereinrichtung (36, 48) ist.

5. Extrakorporale Blutbehandlungsmaschine (1) nach Anspruch 4, **dadurch gekennzeichnet, dass** in der Korrelation (70) eine mittels der Heißdesinfektion (H) zu erreichende Ziel-Letalität (A0) wenigstens eines potentiell in dem Fluidkreislauf (5) vorhandenen Keimes, vorzugsweise fest, abgelegt ist, und dass die Förderdauer (th) mit Erreichen, vorzugsweise Überschreiten, der Ziel-Letalität (A0) endet.

6. Extrakorporale Blutbehandlungsmaschine (1) nach Anspruch 5, **dadurch gekennzeichnet, dass** die Steuereinheit (68) vorgesehen und ausgestaltet ist, die Heizeinrichtung (28) zum Aufheizen des Fluids anzusteuern, bis die Heißdesinfektionstemperatur (Th) stromabwärts der Heizeinrichtung (28) erreicht, insbesondere erfasst wird, und mit Erreichen, insbesondere Erfassen, der Heißdesinfektionstemperatur (Th) die Fördereinrichtung (36, 48) anzusteuern, das Fluid zu fördern, bis die Förderdauer (th), und damit die Ziel-Letalität (A0), erreicht, vorzugsweise überschritten, ist, und danach die Fördereinrichtung (36, 48) anzusteuern, das Fluid nicht mehr zu fördern.

7. Extrakorporale Blutbehandlungsmaschine (1) zumindest nach Anspruch 2 oder 3, **dadurch gekennzeichnet, dass** der wenigstens eine, temperaturabhängige Maschinenprozess ein Testabgleich (T) von wenigstens zwei (60, 62) der Temperatur-Erfassungseinheiten (32, 46, 52, 54, 60, 62) ist, die insbesondere an demselben Abschnitt (50, 58) oder an benachbarten Abschnitten des Fluidkreislaufs (5) angeordnet sind, die höchste oder zu erreichende Prozess-Temperatur (Tp) eine Test-Temperatur (Tt) ist und die Laufzeit (tp) eine Heizdauer (tt) der Heizeinrichtung (28) ist.

8. Extrakorporale Blutbehandlungsmaschine (1) nach Anspruch 7, **dadurch gekennzeichnet, dass** in der Steuereinheit (68) eine Grenztemperatur (Tg) abgelegt ist und die Steuereinheit (68) vorgesehen und ausgestaltet ist, den Mittelwert (Tx) mit der Grenztemperatur (Tg) zu vergleichen, wobei für Mittelwerte (Tx) oberhalb der Grenztemperatur (Tg) in der Steuereinheit (68) die Test-Temperatur (Tt) mit vergleichsweise niedrigem Wert abgelegt ist und für Mittelwerte (Tx) unterhalb der Grenztemperatur (Tg) in der Steuereinheit (68) die Test-Temperatur (Tt) mit vergleichsweise hohem Wert abgelegt ist.

9. Extrakorporale Blutbehandlungsmaschine (1) zumindest nach Anspruch 7, **dadurch gekennzeichnet, dass** die Steuereinheit (68) vorgesehen und ausgestaltet ist, vor Erreichen der Test-Temperatur (Tt) und/ oder vor Ablauf der Heizdauer (tt), die hydraulische Fördereinrichtung (36, 48) anzusteuern, das Fluid beim Aufheizen nicht zu fördern und mit Erreichen der Test-Temperatur (Tt) und/ oder mit Ablauf der Heizdauer (tt), insbesondere mit Ablauf des Aufheizens, die hydraulische Fördereinrichtung (36, 48) anzusteuern, das Fluid zu fördern.

10. Extrakorporale Blutbehandlungsmaschine (1) zumindest nach Anspruch 7, **dadurch gekennzeichnet, dass** die Steuereinheit (68) vorgesehen und ausgestaltet ist, eine Differenz (Td) der von den wenigstens zwei Temperatur-Erfassungseinheiten (52, 54, 60, 62) erfassten Temperaturen (T3, T4, T5, T6) zu ermitteln und wenn die ermittelte Differenz (Td) einen vorbestimmten Wert unterschreitet, einer Ausgabeeinheit der Blutbehandlungsmaschine (1) ein positives Testergebnis der wenigstens zwei Temperatur-Erfassungseinheiten (52, 54, 60, 62) zur Ausgabe bereitzustellen, und ansonsten ein negatives Testergebnis.

11. Verfahren für wenigstens einen temperaturabhängigen Maschinenprozess (H, T) für eine oder einer extrakorporalen Blutbehandlungsmaschine (1), insbesondere Dialysemaschine (1), die vorzugsweise gemäß einem der vorhergehenden Ansprüche ausgebildet ist, wobei die Blutbehandlungsmaschine (1) zumindest aufweist:
- einen Fluidkreislauf (5), insbesondere einen Dialysierflüssigkeitskreislauf,
- eine Heizeinrichtung (28), die vorgesehen und ausgestaltet ist, ein Fluid des Fluidkreislaufs (5), insbesondere Reinstwasser, Primingflüssigkeit oder Desinfektionsflüssigkeit, zur Ausführung des Maschinenprozesses (H, T) aufzuheizen,
- an Abschnitten (30, 38, 50, 58) des Fluidkreislaufs (5) angeordnete Temperatur-Erfassungseinheiten (32, 46, 52, 54, 60, 62), die vorgesehen und ausgestaltet sind, eine jeweilige Abschnitts-Temperatur (T1, T2, T3, T4, T5, T6) des Fluids zu erfassen,
- eine hydraulische Fördereinrichtung (36, 48), die vorgesehen und ausgestaltet ist, das Fluid wahlweise zu fördern oder nicht zu fördern, und
- eine Steuereinheit (68), in der eine Korrelation (70) einer Laufzeit (tp) des wenigstens einen temperaturabhängigen Maschinenprozess (H, T) zumindest in Abhängigkeit von einer Umgebungstemperatur (Tu) abgelegt ist, **gekennzeichnet durch Schritte:**
- Bilden (S2) eines Mittelwertes (Tx) der erfassten Abschnitts-Temperaturen (T1, T2, T3, T4, T5, T6) über die Steuereinheit (68),
- Bestimmen (S3) der Umgebungstemperatur (Tu) in Abhängigkeit des Mittelwertes (Tx), über die Steuereinheit (68), und
- Bestimmen (S5) einer Laufzeit (tp) des Maschinenprozesses (H, T) mittels der Korrelation (70) in Abhängigkeit der bestimmten Umgebungstemperatur (Tu), über die Steuereinheit (68).

12. Verfahren nach Anspruch 11, **dadurch gekennzeichnet, dass** der wenigstens eine temperaturabhängige Maschinenprozess eine Heißdesinfektion (H) zumindest des Fluidkreislaufs (5) ist, wobei in den Schritt Bestimmen (S5) der Laufzeit (tp) der Heißdesinfektion (H) eine Aufheizzeit des Fluids und/oder eine Abkühlzeit des Fluids zumindest anteilig eingeht oder eingehen.

13. Verfahren nach Anspruch 11 oder 12, wobei der wenigstens eine temperaturabhängige Maschinenprozess eine Heißdesinfektion (H) zumindest des Fluidkreislaufs (5) ist, wobei in der Korrelation (70) für die Heißdesinfektion (H) abgelegt sind:
- als Prozess-Temperatur eine Heißdesinfektions-Temperatur (Th), vorzugsweise fest abgelegt,
- eine Ziel-Letalität (A0) wenigstens eines potentiell in dem Fluidkreislauf (5) vorhandenen Keimes, vorzugsweise fest abgelegt, und
- als Laufzeit (tp) eine Förderdauer (th) der hydraulischen Fördereinrichtung (36, 48) in Abhängigkeit der bestimmten Umgebungs-Temperatur (Tu), **gekennzeichnet durch Schritte:**
- Ansteuern (S6.1) der Heizeinrichtung (28) zum Aufheizen des Fluids, über die Steuereinheit (68),
- Ansteuern (S6.2) der Fördereinrichtung (36, 48), das Fluid zu fördern, spätestens, wenn die Heißdesinfektions-Temperatur (Th) stromabwärts der Heizeinrichtung (28) erfasst wird, über die Steuereinheit (68), und
- Ansteuern (S6.3) der Fördereinrichtung (36, 48), das Fluid nicht mehr zu fördern, wenn die Förderdauer (th), und damit die Ziel-Letalität (A0), erreicht ist, vorzugsweise überschritten ist, über die Steuereinheit (68).

14. Verfahren nach Anspruch 11, wobei der wenigstens eine temperaturabhängige Maschinenprozess ein Testabgleich (T) von wenigstens zwei (52, 54, 60, 62) der Temperatur-Erfassungseinheiten (32, 46, 52, 54, 60, 62) ist, die an demselben Abschnitt (50, 58) oder an benachbarten Abschnitten des Fluidkreislaufs (5) angeordnet sind, wobei in der Korrelation (70) für den Testabgleich (T) abgelegt sind:
- eine Grenztemperatur (Tg) der Umgebungstemperatur (Tu),
- für Umgebungstemperaturen (Tu) unterhalb der Grenztemperatur (Tg) eine obere Test-Temperatur (Tto),
- für Umgebungstemperaturen (Tu) oberhalb der Grenztemperatur (Tg) eine untere Test-Temperatur (Ttu), und
- als Laufzeit (tp) eine Heizdauer (tt) der Heizeinrichtung (28), **gekennzeichnet durch Schritte:**
- Vergleichen (S4.1) der bestimmten Umgebungstemperatur (Tu) mit der Grenztemperatur (Tg), über die Steuereinheit (68),
- Bestimmen (S4.2) der oberen Test-Temperatur (Tto), falls die bestimmte Umgebungstemperatur (Tu) kleiner als die Grenztemperatur (Tg) ist, über die Steuereinheit (68),
- Bestimmen (S4.3) der unteren Test-Temperatur (Tto), falls die bestimmte Umgebungstemperatur (Tu) größer als die Grenztemperatur (Tg) ist, über die Steuereinheit (68),
- Bestimmen (S4.4) einer Differenz (ΔT) der bestimmten Test-Temperatur (Tto, Ttu) zur bestimmten Umgebungstemperatur (Tu), über die Steuereinheit (68), die in den Schritt Bestimmen (S5) der Heizdauer (tt) des Testabgleichs (T) mittels der Korrelation (70) in Abhängigkeit der bestimmten Umgebungstemperatur (Tu) eingeht.

15. Verfahren nach Anspruch 14, **gekennzeichnet durch Schritte:**
- Ansteuern (S7.1) der Heizeinrichtung (28), das Fluid für die bestimmte Heizdauer (tt) aufzuheizen, über die Steuereinheit (68)
- Ansteuern (S7.2) der hydraulischen Fördereinrichtung (36, 48), das Fluid vor Ablauf der Heizdauer (tt) nicht zu fördern, über die Steuereinheit (68),
- Ansteuern (S7.3) der hydraulischen Fördereinrichtung (36, 48), das Fluid mit Ablauf der Heizdauer (tt) zu fördern, über die Steuereinheit (68),
- Ermitteln (S7.4) einer Differenz (Td) der von den wenigstens zwei Temperatur-Erfassungseinheiten (52, 54, 60, 62) erfassten Temperaturen (T3, T4, T5, T6), über die Steuereinheit (68),
- Bereitstellen (S7.5) eines positiven Testergebnisses an eine Ausgabeeinheit der Blutbehandlungsmaschine (1) im Falle einer hinreichend kleinen Differenz (Td), und ansonsten Bereitstellen (S7.6) eines negativen Testergebnisses an die Ausgabeeinheit, wobei zumindest der Schritt Ermitteln (S7.4) der Differenz(Td) und die Schritte Bereitstellen (S7.5, S7.6) des Testergebnisses vorzugsweise in mehreren Temperaturbereichen und/ oder Zeiträumen wiederholt wird, bevorzugt in einem höheren Temperaturbereich in einem Zeitraum nach Beginn der Förderung des Fluids, sowie in einem niedrigeren Temperaturbereich in einem Zeitraum nach Beenden der Förderung des Fluids.
